Europäisches Patentamt

European Patent Office

Office européen des brevets

(19)

(11) Veröffentlichungsnummer: **0 228 532**
A1

(12) # EUROPÄISCHE PATENTANMELDUNG

(21) Anmeldenummer: 86115178.5

(22) Anmeldetag: 01.11.86

(51) Int. Cl.⁴: **A 61 B 17/12**

(30) Priorität: 02.11.85 DE 3539022

(43) Veröffentlichungstag der Anmeldung:
15.07.87 Patentblatt 87/29

(84) Benannte Vertragsstaaten:
ES GR

(71) Anmelder: Aigner, Karl, Dr.
c/o Kreiskrankenhaus Trostberg Siegerthöhte 1 Postfach 1165
D-8223 Trostberg(DE)

(72) Erfinder: Aigner, Karl, Dr.
c/o Kreiskrankenhaus Trostberg Siegerthöhte 1 Postfach 1165
D-8223 Trostberg(DE)

(74) Vertreter: Sternagel, Hans-Günther, Dr. et al,
Patentanwälte Dr. Michael Hann Dr. H.-G. Sternagel
Sander Aue 30
D-5060 Bergisch Gladbach 2(DE)

(54) Implantierbarer Katheter mit Gefässdrossel.

(57) Implantierbarer Katheter mit einer an der Spitze des Katheterrohres (1) als Gefäßdrossel ausgebildeten Klammer (11), deren Arme (13, 15) an einem Ende schwenkbar miteinander verbunden sind, und deren andere Enden in Kontakt miteinander gebracht werden können, so daß zwischen den Armen ein Raum vorhanden ist, wobei einer Arme (13) auf der Innenseite einen das Ende des Katheterrohres (1) bildenden aublasbaren Ballon (12) aufweist, der in aufgeblasenem Zustand den Zwischenraum zwischen den Armen (13, 14) der Klammer ausfüllt. Von den nicht miteinander verbundenen Enden der Arme (13, 14) der Klammer (11) ragen nach außen abgebogene Stege (17, 17a) ab, wobei am Steg (17) ein Einrastzapfen angeordnet ist, der in eine Bohrung am Steg (17a) eingreift und eine Verriegelung der Klammerarme miteinander ermöglicht.

Fig.1

EP 0 228 532 A1

BESCHREIBUNG:

Die Erfindung bezieht sich auf einen implantierbaren Katheter, dessen Katheterspitze als Gefäßdrossel ausgebildet ist, und bei dem am proximalen Ende des Katheterrohres ein Zuspritzteil angeordnet ist, von dem aus die Drossel aktiviert werden kann.

Für gewisse Therapien ist es erstrebenswert, eine synchron zur Direktzufuhr von Pharmazeutika oder Hilfsstoffen in Blutgefäße erfolgende intermetierende Drosselung des Blutflusses des zuführenden Blutgefäßes zu ermöglichen und ggfs. über eine totale Blockade des Blutstromes im Organ über beliebige Zeitintervalle steuerbar zur machen. Dies ist beispielsweise bei der zytostatischen Chemotherapie besonders erwünscht. Dadurch wird eine längere Einwirkzeit der zugeführten Zytostatika und damit ein höherer Tumorzytostatikaspiegel erreicht.

Es sind verschiedene Techniken bekannt, den Blutdurchfluß vorübergehend oder bleibend zu reduzieren, wie Ligatur oder die Verwendung intravasaler Ballon-Katheter oder der Einsatz von vasokonstriktiven Medikamenten.

Eine dauerhafte Verlegung eines Blutgefäßes, insbesondere von Arterien, gefährdet die Versorgung des betreffenden Organs und führt zur sekundären Arterialisation. Intravasale Ballon-Katheter können gegebenenfalls durch Thrombusbildung das Gefäß ebenfalls bleibend blockieren.

Teilerfolge brachte der Einsatz von sogenannten Microsphären (Stärkepartikel von etwa 40 μ Größe), welche temporär den kapilllaren Bereich im Organ blockieren und damit den Blutdurchfluß verlangsamen bzw. stoppen. Dieses Hilfsmittel ist aber schon bereits nach 10 bis 15 Minuten so weit abgebaut, daß eine ausreichende Blockade des Blutflusses nicht mehr möglich ist.

Aufgabe der vorliegenden Erfindung ist es, einen Katheter zu schaffen, der eine kontrollierte Drosselung des Durchflusses in Blutgefäßen bis zur völligen Blockade ermöglicht und der nicht nur im offenen Operationsfeld verwendet werden kann, sondern auch dauerhaft implantierbar ist.

Diese Aufgabe wird gelöst durch einen implantierbaren Katheter mit einer Katheterspitze, einem sich daran anschließenden Katheterrohr und einem an dessen der Katheterspitze abgewandten Ende angeordneten Zuspritzteil in Form einer runden Schale, deren Oberseite mit einer elastischen Membran dicht verschlossen ist, der dadurch gekennzeichnet ist, daß die Katheterspitze als Gefäßdrossel ausgebildet ist in Form einer am Katheterrohr befestigten Klammer, deren Arme an einem Ende schwenkbar miteinander verbunden sind, und deren andere Enden in Kontakt miteinander gebracht werden können, so daß zwischen den Armen ein Raum vorhanden ist, wobei einer der Arme auf der Innenseite einen das Ende des Katheterrohres bildenden aufblasbaren Ballon aufweist, der in aufgeblasenem Zustand den Zwischenraum zwischen den Armen der Klammer ausfüllt. Vorzugsweise sind die Arme der Klammer gegeneinander gebogen. Ganz besonders bevorzugt ist eine Ausführungsform der Klammer mit einem flachen Arm und einem halbkreisförmigen Arm, die aneinander gelenkt sind, und wobei der aufblasbare Ballon auf der Klammerinnenseite des flachen Armes angeordnet ist. Grundsätzlich ist auch eine Anordnung und Befestigung des Ballons an der Innenseite des gebogenen Armes möglich.

Die in Kontakt miteinander bringbaren Enden der Arme der Klammer werden durch ein Schnappschloß in festem Kontakt gehalten. Bei einer anderen Ausführungsform der Drossel werden die in Kontakt miteinander bringbaren Enden der Arme durch an der Verbindungsstelle der anderen Enden der Arme befestigte und auf die Arme einwirkende Federn in festem Kontakt gehalten.

Der oder die gebogenen Arme weisen an ihren freien Enden jeweils einen nach außen abgebogenen Steg auf, der bei geschlossener Klammer auf dem Steg des anderen Armes aufliegt. Um einen Schnappverschluß auszubilden, ist am Steg des einen Armes auf der dem anderen Arm zugewandten Seite ein vom Steg abstehender Einrastzapfen angeordnet, der in eine fluchtende Bohrung im Steg des gegenüberliegenden Armes eingreift. Zur Sicherung des Verschlusses ist es besonders vorteilhaft, wenn die Stege seitlich von dem Verschlußzapfen noch jeweils in der Nähe der seitlichen Außenkanten jeweils zwei Bohrungen aufweisen, wobei die Bohrungen der sich gegenüberliegenden Stege miteinander fluchten.

Dies ermöglicht die Verschlußsicherung durch Fäden und auch eine räumliche Fixierung der Gefäßdrossel im Patienten.

Die Klammer kann am distalen Ende des Katheterrohres auch über eine verriegelbare Kegelverbindung nach DIN 13 090 verbunden sein, um einen Austausch der Klammer ohne Wechsel des Katheterrohres zu ermöglichen. In einem solchen Falle weist die Klammer einen Anschlußstutzen für die Kegelverbindung auf, und auf der Innenseite des Klammerarmes ist der aufblasbare Ballon befestigt, dessen Inneres in Verbindung mit der Innenbohrung des Anschlußstutzens steht.

Zur besseren Fixierung des Zuspritzteils im subkutanen Bereich der Haut weist die Schale des Zuspritzteils am proximalen oder hinteren Ende des Kahteterrohres an der Unterseite einen seitlich überstehenden Rand auf. Der obere Schalenrand ist als ein über die Membran überstehender Randwulst ausgebildet, um die Injektionsfläche besser ertasten zu können. Das Zuspritzteil ist mit dem Katheterrohr durch eine an einem seitlich am Zuspritzteil angeordneten Anschlußstutzen angreifende Steckverbindung verbunden. Dies ermöglich einen Austausch des Zuspritzteils, ohne einen Wechsel des Katheterrohres mit der tiefer liegenden Drossel, wenn dies nach wiederholten Injektionen durch die elastische Membran erforderlich sein sollte.

Der Ballon kann sowohl pneumatisch oder hydraulisch mittels Gasen oder physiologisch verträglichen Flüssigkeiten aufgeweitet werden. Die Druckgabe und Druckentlastung geschieht dabei mittels einer Injektionsspritze oder Injektionskanüle perkutan über das implantierbare Zuspritzteil. Wenn die Gefäßdrossel sich im geöffneten Zustand befindet, d.h. die in Kontakt bringbaren Enden der Klammerarme einen Abstand voneinander aufweisen, kann die Klammer um ein Blutgefäß angeordnet werden. Nach Verschluß der Klammer befindet sich das Blutgefäß im Inneren der Klammer, und der Durchfluß wird durch den aufgeweiteten Ballon je nach Ballonvolumen für die gewünschte Dauer beliebig gedrosselt werden.

Der aufblasbare Ballon besteht aus synthetischem oder natürlichem Kautschuk. Die Klammer der Gefäßdrossel kann aus Metall sein, bevorzugt sind aber Kunststoffe wie Polyurethan, medizinisch verträgliches PVC, Acrylharze, Polycarbonat oder Polyamid. Für das Katheterrohr sind besonders elastische Kunststoffe geeignet, beispielsweise Polyurethane, medizinisch verträgliches PVC, Polyamide oder Silikone. Die Schale des Zuspritzteils wird aus Kunststoff gefertigt, vorzugsweise Polyamid, Polyurethan oder Polyoxymethylen. Als besonders geeignet für die Membran haben sich Siliconkautschuk oder Naturkautschuk erwiesen. Geeignet sind aber auch andere synthetische Polymere, sofern sie eine ausreichende Elastizität aufweisen und vielfaches Perforieren erlauben.

Um die Gewebeverträglichkeit zu verbessern, hat es sich als besonders vorteilhaft herausgestellt, sowohl das Zuspritzteil einschließlich der Membran als auch ggfs. die Gefäßdrossel mit einer porösen Kunststoffschicht zu überziehen oder einen Film aufzubringen. Dafür haben sich als besonders gewebeverträgliche Kunststoffe bewährt Polyacrylnitril und andere Polyacrylharze, Polyamid, Polyurethane, Polyäthylenglykolterephthalat oder Silikone. Polyäthylenglykolterephthalat oder eine Mischung aus Polyurethan und Silikon sind bei Verwendung von elastischen Membranen aus Naturkautschuk besonders bevorzugt.

Um neben der Drosselung des Durchflusses durch ein Blutgefäß gleichzeitig auch die gewünschten Chemotherapeutika zuführen zu können, ist die Mitverwendung eines implantierbaren sogenannten Zuspritzkatheters erforderlich. Ein solcher Zuspritzkatheter ist in DE-OS-33 09 788 beschrieben. Für die Kombination des Drosselkatheter mit einem Zuspritzkatheter ist es besonders günstig, wenn das Zuspritzteil des implantierbaren Drosselkatheters auf einer implantierbaren Kunststoffplatte angeordnet ist und neben dem Zuspritzteil für den die Gefäßdrossel aufweisenden Katheter noch ein Zuspritzteil für einen Zuspritzkatheter angeordnet ist, dessen Katheterrohr kurz vor seinem distalen Ende einen Außenwulst aufweist, um das Herausrutschen der Katheterspitze aus dem betreffenden Blutgefäß zu verhindern. Dieser zusätzlich vorhandene Zuspritzkatheter weist am distalen Ende des Katheterrohres vorzugsweise ein Rückschlagventil auf.

Der aus DE-OS 33 09 788 bekannte implantierbare Zuspritzkatheter mit einem Katheterrohr und einem an dessen der Katheterspitze abgewandeten Ende angeordneten Zuspritzteil in Form einer runden Schale, deren Oberseite mit einer elastischen Membran als Injektionsplatte dicht verschlossen ist und die an der Unterseite einen seitlich überstehenden Rand aufweist, unterscheidet sich vom Drosselkatheter nur dadurch, daß an der Katheterspitze anstelle der als Gefäßdrossel dienenden Klammer in der Nähe der offenen Katheterspitze lediglich ein Außenwulst vorhanden ist oder daß die Katheterspitze mit einem Rückschlagventil versehen ist. Die konstruktive Gestaltung des Zuspritzteils ist identisch mit der Form des Zuspritzteils für den Drosselkatheter.

Wie bereits beschrieben ist es besonders bevorzugt, die beiden Zuspritzteile auf einer dünnen elastischen Kunststoffplatte nebeneinander anzubringen, wobei diese Platte dann in der Nähe ihres abgerundeten Randes Bohrungen aufweist, die eine Fixierung der Platte im subkutanen Hautbereich ermöglicht.

Die erfindungsgemäße konstruktive Gestaltung des Occlusions- oder Gefäßdrosselkatheters in Kombination mit einem implantierbaren Zuspritzkatheter ermöglicht es, bei der intraarteriellen zytostatischen Chemotherapie die Zytostatikakonzentration im Tumorgewebe zu erhöhen und gleichzeitig die systemische Wirkung zu erniedrigen. Dies hat eine Verrringerung der Nebenwirkungen und damit bessere Lebensqualität zur Folge. Die intraarterielle Chemotherapie weist gegenüber der intraavenösen systemischen Zufuhr der Chemotherapeutika Vorteile auf. Die Gewebekonzentration ist dabei abhängig von der Gesamtdosis, der Injektionsgeschwindigkeit, der Blutdurchflußrate über die infundierte Arterie in den Tumor und der Austauschgeschwindigkeit der verwendeten Substanz zwischen den Verteilungsräumen im Tumor sowie der Eliminationsgeschwindigkeit des infundierten Medikamtes im Organismus. Der erfindungsgemäße Katheter ermöglicht es, im interstitiellen bzw. intrazellulären Raum eine niedrige BLutdurchflußrate zumindest für die Dauer der Perfusion des Tumorbereiches einzustellen, indem synchron zur Zytostatikazufuhr eine intermetierende Drosselung des Blutflusses der zuführenden Arterie mittels der Gefäßdrossel erfolgt. Es ist dabei möglich, eine totale Blockade des Blutstromes im Organ über beliebige Zeitintervalle zu steuern. Je nach eingesetztem Zytostatikum kann die Blockadedauer variiert werden, wobei Langzeitunterbrechungen des Blutflusses (von einer halben bis zu mehreren Stunden), wie sie mit speziellen Mikrosphären erreicht werden, vermieden werden können, da sie die Ausbildung einer zusätzlichen arteriellen Blutversorgung des Tumors aus sekundären Quellen induzieren und damit langfristig den isolierten Zugang für Zytostatika unmöglich machen. Die steuerbare Gefäßocclusion wird erreicht durch die eine die betreffende Arterie umfassende Klammer mit dem aufblasbaren Ballon. Der Ballon kann über eine gewünschte Dauer den Blutdurchfluß drosseln, danach aber durch Aspiration des druckgebenden Mediums wieder freigeben.

Als beispielhaft werden folgende Anwendungsbereiche genannt:

1. Arteria hepatica Tourniquet-Infusion:
   Dabei wird die Drossel um den Hauptstamm der Arteria hepatica gelegt. Durch perkutane Punktion des Zuspritzteils und Katheter-Reservoirs wird der Ballon der Gefäßdrossel mit Gas oder Flüssigkeit gefüllt, so daß die Leberarterie verschlossen wird. Gleichzeitig wird über einen leberwärts implantierten Zuspritzkatheter während der Drosselung des Durchflusses Zytostatika in voller Dosis und Konzentration in die Leber bzw. den Tumor befördert und eine längere Einwirkzeit bei höherem Tumorzytostatikaspiegel erreicht.

2. Modifizierung der Extremitätenperfusion:
   Durch vorrübergehende Blockierung der Arterie und Vene in der Leiste wird der Blutfluß im Bein gestoppt bzw. verringert, und es kann selektiv Zytostatikum in den Ober- oder Unterschenkel zur Behandlung von Melanomen oder Weichteiltumoren gegeben werden.

3. Bei Tumoren im kleinen Bedcken dient der Drosselkatheter zur Blockierung der Arterica iliaca interna, und die Infusion erfolgt arteriell über den mit implantierten Zuspritzkatheter.

4. Generell kann der erfindungsgemäße Drosselkatheter in der Gefäßchirurgie angewendet werden, wenn an unwegsamen Stellen, an denen herkömmliche Gefäßklemmen behindernd wirken, vorübergehend der Blutfluß unterbrochen werden muß. In einem solchen Falle werden bevorzugt Ausführungsformen des erfindungsgemäßen Katheters verwendet, die nicht mit zusätzlichen gewebekompatiblen Kunststoffschichten überzogen sind, weil das erleichterte Einwachsen von Gewebe nicht erforderlich ist.

Die Erfindung wird nun anhand der Figuren noch näher erläutert.

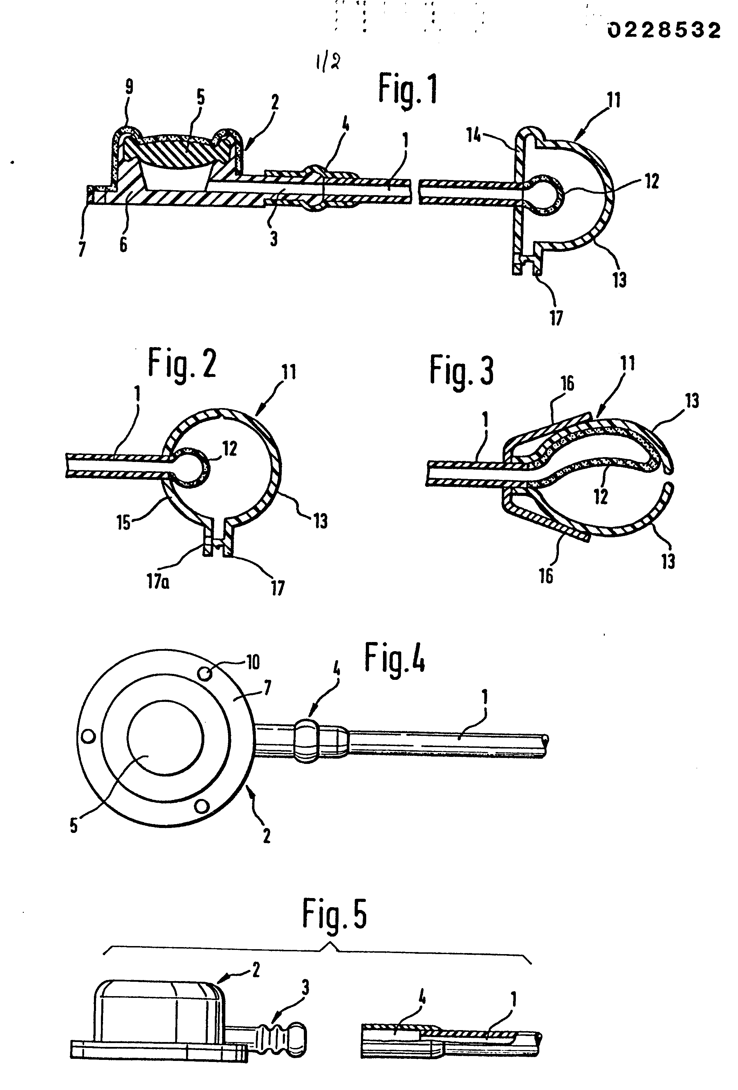

Figur 1 zeigt den implantierbaren Katheter mit einer Gefäßdrossel als Katheterspitze im Längsschnitt. Die als Gefäßdrossel ausgebildete Katheterspitze weist eine am Katheterrohr 1 befestigte Klammer 11 mit zwei Armen 13, 14 auf, wobei der Arm 13 mit dem Arm 14 durch ein Schnappschloß verriegelt werden kann. An einem der Klammerarme 14 ist auf der Innenseite ein, das Ende des Katheterrohres 1 bildender aufblasbarer Ballon 12 angeordnet. Der Ballon 12 füllt in aufgeblasenem Zustand den Zwischenraum zwischen den Armen 13, 14 der Klammer 11 aus.

Am anderen Ende des Katheterrohres 1 ist ein Zuspritzteil 2 angeordnet. Die Druckgabe und Druckentlastung des Ballons 12 geschieht mittels einer Injektionsspritze oder Injektionskanüle perkutan über das Zuspritzteil 2. Eingebrachte Flüssigkeit oder Gas gelangt durch das Katheterrohr 1 in den Ballon 12, so daß zur Gefäßdrosselung Druck über das Katheterrohr 1 in den Ballon 12 der Gefäßdrossel weitergegeben wird, so daß sich das Volumen des Ballons vergrößert. Der aufgeblasene Ballon 12 drückt gegen die Wand eines durch den Zwischenraum zwischen den Klammerarmen 13, 14 geführten Blutgefäßes und drosselt je nach Volumen des Ballons den Blutdurchfluß. Grundsätzlich ist eine vollständige Sperrung des Gefäßes durch einen entsprechend stark aufgeweiteten Ballon möglich.

Das Zuspritzteil 2 weist einen seitlichen Anschlußstutzen 3 auf, an den mittels einer Steckverbindung 4 das Katheterrohr 1 angeschlossen ist. Dies ermöglicht bei Bedarf ein Auswechseln entweder des Zuspritzteils 2 oder des Katheterrohres 1 mit der Gefäßdrossel 11.

Das Zuspritzteil 2 hat die Form einer Dose oder flachen Schale 6, deren Oberseite mit einer elastischen Membran 5 verschlossen ist, die in den nach oben gerichteten Rand der Schale 6 dicht eingefügt ist. Der obere Schalenrand des Zuspritzteils 2 ist als ein über die Membran 5 überstehender Randwulst ausgebildet. Zur besseren Fixierung des Zuspritzteils 2 im Körper eines Patienten ist auf der Unterseite der Schale ein seitlich überstehender Rand 7 vorgesehen. Die zur Verbesserung der Gewebeverträglichkeit vorgesehene Beschichtung der Oberfläche der elastischen Membran 5 und des Zuspritzteils 2 mit einem gewebskompatiblen porösen Kunststoff ist in Figur 1 mit Bezugszeichen 9 angedeutet, wobei die Schichtdicke zur Verdeutlichung stark übertrieben ist. Das Katheterrohr 1 ist vorzugsweise 200 - 500 mm lang, weist einen Innendurchmesser von 0,5 bis 1,5 mm und einen Außendurchmesser von 1 bis 3 mm auf.

Figur 2 zeigt im Längsschnitt eine andere Ausführungsform der als Gefäßdrossel dienenden Klammer 11. Auch in diesem Falle steht das Ende des Katheterrohres 1 in Verbindung mit dem Inneren des aufblasbaren Ballons 12 am Ende des Katheterrohres 1. Die am Ende des Katheterrohres 1 befestigte Klammer 11 weist zwei halbkreisförmig gegeneinander gebogene Klammerarme 13, 15 auf, die an einer Seite miteinander verbunden sind. Die Verbindung kann durch eine geringere Materialstärke schwenkbar ausgebildet sein oder durch eine übliche Anlenkung eine Öffnung der Klammerhälften ermöglichen. An den anderen Enden der Klammerarme 15, 13 sind seitlich nach außen abstehende Stege 17, 17a ausgebildet, die parallel zueinander verlaufen und bei geschlossener Klammer miteinander in Kontakt stehen. Der Steg 17 weist auf der dem Steg 17a zugewandten Seite einen Einrastnippel auf, der in eine im Steg 17a vorhandene Bohrung eingreift, so daß die Klammer 11 verriegelbar ist.

Figur 3 zeigt eine andere Ausführungsform der Klammer 11 am Ende des Katheterrohres 12 im Längsschnitt. Die beiden Klammerarme 13 sind gegeneinander gebogen und an der Innenfläche eines der Arme ist der mit dem Katheterrohr 1 in Verbindung stehende aufblasbare Ballon 12 befestigt. Die beiden Klammerarme 13 werden durch den Druck der Federn 16, die auf die Außenseite der Klammerarme 13 einwirken, und an dem Ende, an dem die Klammerarme 13 aneinander angelenkt sind, befestigt sind. Figur 3 zeigt schematisch die Anordnung der Klammer 16.

Figur 4 zeigt das Zuspritzteil 2 von oben, wobei der sich nach außen erstreckende seitliche Rand 7 mit Löchern 10 zum Fixieren mittels Fäden besonders deutlich zu erkennen ist. Im Zentrum des Zuspritzteils 2 auf der Oberseite liegt die Injektionsplatte in Form einer elastischen Membran 5. An den seitlich abführenden Stutzen ist mit der Steckverbindung 4 das Katheterrohr 1 angeschlossen.

Figur 5 zeigt das Zuspritzteil 2 in vom Katheterrohr 1 abgetrennten Zustand. Der seitliche Anschlußstutzen 3 am Zuspritzteil 2 weist an seiner Oberfläche Kerben und an seinem Ende einen Wulst auf, so daß das als übergreifende Hülse ausgebildete Ende des Katheterrohres 1 darüber geschoben werden kann und die Steckverbindung 4 bildet. Der Endwulst und die Rippen auf der Oberfläche des Stutzens 3 ergeben einen dichten, festen Sitz der Hülse des Katheterrohres und bewirken eine dauerhafte und dichte Verbindung, die jedoch im Bedarfsfalle lösbar ist und das Anschließen eines neuen Zuspritzteils 2 an die Steckverbindung 4 oder den Anschluß eines neuen Katheterrohres 1 mit Steckverbindung 4 an den Anschlußstutzen 3 des Zuspritzteils 2 ermöglicht. Das Katheterrohr 1 wird vorzugsweise aus Siliconkautschuk oder Polyurethan hergestellt. Dieses Material ist ausreichend elastisch, um eine dichte Steckverbindung zu ermöglichen, es können jedoch auch andere gewebeverträgliche Kunststoffe für das Katheterrohr 1 verwendet werden.

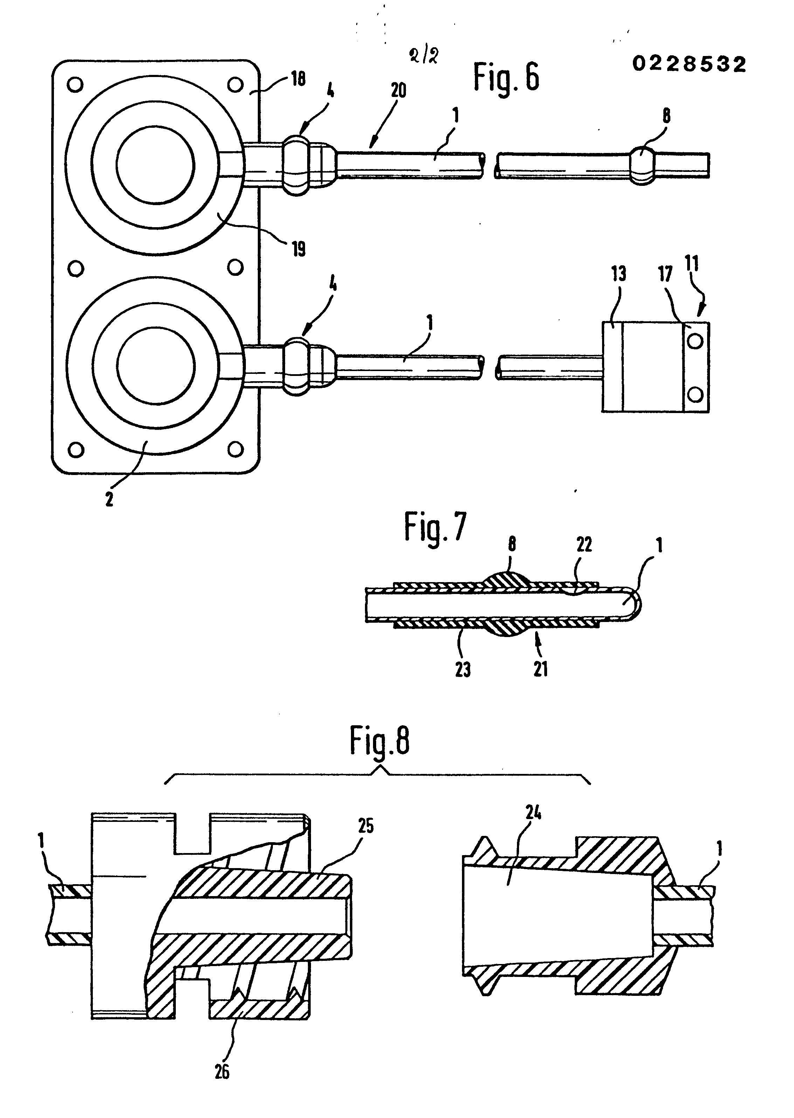

Figur 6 zeigt die Kombination des erfindungsgemäßen Katheters mit Gefäßdrossel mit einem Zuspritzkatheter, der ebenfalls implantiert werden kann. Die beiden Zuspritzteile 2 und 19 sind auf einer dünnen Kunststoffplatte 18 befestigt, und die Anschlußstutzen mit den Steckverbindungen 4 für die Katheterrohre 1 ragen über den Rand der Platte 18 hinaus. Am Ende des einen Katheterrohres 1 ist die als Gefäßdrossel ausgebildete Klammer 11 angeschlossen. Die Klammerarme 13 weisen an den abstehenden Stegen 17 je zwei in der Nähe der seitlichen Kanten angeordnete miteinander fluchtene Bohrungen auf, die es ermöglichen sollen, mittels Fäden, die Position der Gefäßdrossel im Patienten zu fixieren. Außerdem ermöglichen Fäden die zusätzliche Sicherung des Schnappverschlusses.

Am zweiten Zuspritzteil 19 mit dem Katheterrohr 1 ist an der Spitze des Katheterrohres in Abstand von der Spitze ein Außenwulst 8 vorhanden, um das Herausgleiten des Katheterrohres aus einem Blutgefäß zu verhindern.

Vorzugsweise weist das Katheterrohr 1 des eigentlichen Zuspritzkatheters 20 an der Spitze ein Rückschlagventil 21 auf, wie es beispielsweise in Figur 7 im Längsschnitt wiedergegeben ist. Die eigentliche Spitze des Katheterrohrs 1 ist verschlossen. Das Katheterrohr weist zwei gegeneinander versetzte seitliche Öffnungen 22 auf, über die ein elastischer Ventilschlauch 23 aus geeignetem Material gezogen ist und diese verschließt. Dieser Ventilschlauch weist einen Außenwulst 8 auf, der mit dem Außenwulst 8 am Katheterrohr 1 in Figur 6 in seiner Funktion identisch ist und das Herausgleiten der Katheterspitze aus einem Blutgefäß erschweren soll.

Durch den von Innen wirkenden Injektionsdruck wird der Ventilschlauch gedehnt, und die Flüssigkeit kann aus dem zur Spitze hin offenen Ventilschlauch austreten. Der hintere Teil des Ventilschlauches schließt sich direkt an den Außenwulst an und kann dort auch an der Oberfläche des Katheterrohres befestigt sein. Im Ruhezustand verschließt der Ventilschlauch die Öffnung und läßt den Austritt von Blut in das Katheterrohr nicht zu. Der besondere Vorteil dieser konstruktiven Gestaltung besteht darin, daß der Außenwulst auf dem Katheterrohr eine Arretierung der Katheterspitze im Gefäß ermöglicht, und so die Gefahr des Herausrutschens vermindert ist.

Durch die Ausbildung einer Steckverbindung zwischen Katheterrohr 1 und den Zuspritzteilen 19 und 2, wie sie in Figur 6 wiedergegeben sind, ist ein Austausch der Zuspritzteile bei etwaigem Versagen nach vielen Funktionen möglich, ohne daß eine neue Freilegung der tiefer im Körper liegenden Gefäße erforderlich ist, weil die Katheterrohre nicht ausgetauscht werden brauchen.

Die Beschichtung der Zuspritzteile und der elastischen Membranen und ggfs. auch der Platte 18 mit speziellen Kunststoffen hat den Vorteil, daß diese Teile mit dem Gewebe besser verwachsen können und so eine zusätzliche Abdichtung der bei Injektionen jeweils zu durchstechenden Membran erreicht wird.

In _Figur 8_ ist eine weitere Ausführungsform einer Auftrennung des Katheterrohres wiedergegeben, die vorzugsweise dazu verwendet werden kann, die Gefäßdrossel mit der Klammer 11 austauschbar zu machen, wenn ein Austausch des gesamten Katheterrohres zusammen mit der Drossel vermieden werden soll. Es handelt sich dabei um eine verriegelbare Kegelverbindung nach DIN 13 090, deren ineinander steckbare Elemente am Katheterrohr 1 befestigt sind. Das rechte Teilstück weist einen sogenannten weiblichen Luerkonus 24 auf. Von der Außenseite der Konushülse stehen am vorderen Ende zwei Flügel ab, die Verriegelungselemente in ein Gewinde des Gegenstückes eingreifen. Ein solches Verbindungsteil wird in der DIN-Vorschrift als Verbindungsteil LLS mit Innenkegel bezeichnet. Das Gegenstück ist ein Verbindungsteil LLN mit Außenkegel, d.h. einem einschiebbaren Stutzen 25, der in Abstand von einer Hülse 26 mit Innengewinde umgeben ist.

Die Kegelverbindung wird aus implantierbaren und gewebeverträglichem Kunststoff hergestellt, vorzugsweise aus Polyacrylharzen, Polyamid, Polyurethan oder Polyäthylenglykolterephthalat.

0228532

BEZUGSZEICHENLISTE:

_____

| 1      | Katheterrohr                              |
|--------|-------------------------------------------|
| 2      | Zuspritzteil                              |
| 3      | seitlicher Anschlußstuten                 |
| 4      | Steckverbindung                           |
| 5      | elastische Membran                        |
| 6      | Schale des Zuspritzteils                  |
| 7      | seitlich überstehender Rand               |
| 8      | Außenwulst am Katheterrohr                |
| 9      | Beflockungsschicht                        |
| 10     | Befestigungslöcher                        |
| 11     | Gefäßdrossel, Klammer                     |
| 12     | aufblasbarer Ballon                       |
| 13, 14 | Klammerarme                               |
| 15     | Klammerarm                                |
| 16     | Feder                                     |
| 17     | Steg des Klammerarms                      |
| 17a    | Steg des Klammerarms                      |
| 18     | Kunststoffplatte                          |
| 19     | Zuspritzteil                              |
| 20     | Zuspritzkatheter                          |
| 21     | Rückschlagventil                          |
| 22     | Öffnung im Katheterrohr des Rückschlagventils |
| 23     | Ventilschlauch                            |
| 24     | Verbindungsteil mit Innenkonus            |
| 25     | Verbindungsteil mit Außenkonus            |
| 26     | Gewindehülse                              |

-1-

0228532

PATENTANSPRÜCHE:

1. Implantierbarer Katheter mit einem Katheterrohr und einem an dessen der Katheterspitze abgewandten Ende angeordneten Zuspritzteil in Form einer runden Schale, deren Oberseite mit einer elastischen Membran als Injektionsplatte dicht verschlossen ist, dadurch gekennzeichnet, daß die Katheterspitze als Gefäßdrossel ausgebildet ist in Form einer am Katheterrohr (1) befestigten Klammer (11), deren Arme (13, 15) an einem Ende schwenkbar miteinander verbunden sind, und deren andere Enden in Kontakt miteinander gebracht werden können, so daß zwischen den Armen ein Raum vorhanden ist, wobei einer der Arme (13) auf der Innenseite einen das Ende des Katheterrohres (1) bildenden, aufblasbaren Ballon (12) aufweist, der in aufgeblasenem Zustand den Zwischenraum zwischen den Armen (13, 15) der Klammer (11) ausfüllt.

2. Katheter nach Anspruch 1, dadurch gekennzeichnet, daß die Klammer (11) zwei gegeneinander gebogene Arme (13, 15) aufweist.

3. Katheter nach Anspruch 1, dadurch gekennzeichnet, daß die Klammer (11) einen flachen Arm (14) und einen halbkreisförmigen Arm (13) aufweist, wobei der aufblasbare Ballon (12) auf der Klammerinnenseite des flachen Armes (14) angeordnet ist.

0228532

4. Katheter nach Ansprüchen 1, 2 oder 3,
   d a d u r c h   g e k e n n z e i c h n e t ,
   daß die in Kontakt miteinander bringbaren Enden der
   Arme (13, 15) der Klammer (11) durch ein Schnappschloß
   in festem Kontakt gehalten sind.

5. Katheter nach Ansprüchen 2 oder 3,
   d a d u r c h   g e k e n n z e i c h n e t ,
   daß die in Kontakt miteinander bringbaren Enden der
   Arme (13) der Klammer (11) durch eine an der Verbindungsstelle der anderen Enden der Arme (13) befestigte
   und auf die Arme (13) einwirkende Feder (16) in festem
   Kontakt gehalten sind.

6. Katheter nach Ansprüchen 1, 2, 3 oder 4,
   d a d u r c h   g e k e n n z e i c h n e t ,
   daß der oder die gebogenen Arme an ihren freien Enden
   einen nach außen abgebogenen Steg (17) aufweisen, der
   bei geschlossener Klammer auf dem Steg des anderen
   Armes aufliegt, und der Steg (17) und der Steg (17a)
   des anderen Armes je zwei in Abstand voneinander und
   miteinander fluchtende Bohrungen aufweisen, und
   zwischen den Bohrungen am Steg (17) einer der Arme ein
   Einrastzapfen angeordnet ist, der in eine dritte
   Bohrung am Steg (17a) des anderen Armes (14)
   eingreift.

7. Katheter nach jedem der Ansprüche 1 bis 6,
   d a d u r c h   g e k e n n z e i c h n e t ,
   daß das distale Ende des Katheterrohres (1) mit der den
   aufblasbaren Ballon (12) aufweisenden Klammer (11) über
   eine verriegelbare Kegelverbindung nach DIN 13 090
   verbunden ist.

8. Katheter nach jedem der Ansprüche 1 bis 6, d a d u r c h  g e k e n n z e i c h n e t , daß die Schale des Zuspritzteils (2) an der Unterseite einen seitlich überstehenden Rand (7) aufweist, und der obere Schalenrand als ein über die Membran überstehender Randwulst ausgebildet ist, und daß das Zuspritzteil (2) mit dem Katheterrohr (1) durch eine, an einem seitlich am Zuspritzteil (2) angeordneten Anschlußstutzen (3) angreifende Steckverbindung (4) verbunden ist.

9. Katheter nach Anspruch 1, d a d u r c h  g e k e n n z e i c h n e t , daß der aufblasbare Ballon (12) aus synthetischem oder natürlichem Kautschuk, die Klammer (11) aus Polyurethan, medizinisch verträglichem PVC, Acrylharz, Polyoxymethylen, Polycarbonat oder aus Polyamid besteht.

10. Katheter nach jedem der Ansprüche 1 bis 9, d a d u r c h  g e k e n n z e i c h n e t , daß das Zuspritzteil (2) auf einer implantierbaren Kunststoffplatte (18) angeordnet ist, und neben dem Zuspritzteil (2) für den die Gefäßdrossel aufweisenden Katheter noch ein Zuspritzteil (19) für einen Zuspritzkatheter (20) angeordnet ist, dessen Katheterrohr kurz vor dem distalen Ende einen Außenwulst (8) aufweist.

11. Katheter nach Anspruch 10, d a d u r c h  g e k e n n z e i c h n e t , daß das Katheterrohr (1) des Zuspritzkatheters (20) am distalen Ende ein Rückschlagventil (21) aufweist.

12. Katheter nach jedem der Ansprüche 1 bis 11,
d a d u r c h   g e k e n n z e i c h n e t ,
daß die Oberfläche der elastischen Membranen (5) und
der Zuspritzteile (2 und 19) und gegebenenfalls der
Kunststoffplatte (18) mit einem gewebskompatiblen,
porösen Kunststoff, der das Einwachsen von Zellen begünstigt, beschichtet ist.

**Fig.1**

**Fig. 2**

**Fig. 3**

**Fig.4**

**Fig.5**

Fig. 6

0228532

Fig. 7

Fig. 8

**Europäisches Patentamt**

**EUROPÄISCHER RECHERCHENBERICHT**

Nummer der Anmeldung

**0228532**

EP 86 11 5178

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | EP-A-0 119 596 (K. AIGNER) <br><br> * Zusammenfassung; Figuren 1-3 * | 1-3,8, 9,11, 12 | A 61 B 17/12 |
| Y | US-A-2 533 924 (F.E.B. FOLEY) <br><br> * Spalte 2, Zeilen 34-39; Spalte 3, Zeilen 15-22; Figuren 2-4 * | 1-3,8, 9,11, 12 | |
| A | | 4-6 | |
| A | US-A-3 675 656 (S. HAKIM) <br> * Figuren 1-5; Spalte 1, Zeilen 24-39 * | 1-6,9 | |
| A | US-A-3 538 917 (R.G. SELKER) <br> * Figur 1; Spalte 2, Zeilen 27-33 * | 1 | **RECHERCHIERTE SACHGEBIETE (Int. Cl.4)** <br><br> A 61 B <br> A 61 F <br> A 61 M |
| A | FR-A-2 254 352 (R. MAILLET) <br><br> * Figur 5 * | | |
| A | US-A-4 512 766 (V.L. VAILANCOURT) <br> * Figur 1 * | 7 | |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 12-02-1987 | RAKOWICZ, J.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

## Europäisches Patentamt

# EUROPÄISCHER RECHERCHENBERICHT

Nummer der Anmeldung

# 0228532

EP 86 11 5178

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP-A-0 073 655  (ETHICON INC.) <br> * Figuren 1-3 * | 6 | |
| A | US-A-4 227 533  (J.C. GODFREY) <br> * Figuren 1,2 * | 11 | |
| A | GB-A-1 471 609  (DOWNS SURGICAL LTD) <br> * Figur 1 * | 10 | |
| | ----- | | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| DEN HAAG | 12-02-1987 | RAKOWICZ,J.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82